Europäisches Patentamt

European Patent Office

Office européen des brevets

(11) Publication number: **0 419 071 A2**

# EUROPEAN PATENT APPLICATION

(21) Application number: 90309544.6

(22) Date of filing: 31.08.90

(51) Int. Cl.⁵: **A61B 17/068**

(30) Priority: 31.08.89 GB 8919691

(43) Date of publication of application:
27.03.91 Bulletin 91/13

(84) Designated Contracting States:
DE FR IT

(71) Applicant: TAYLOR, James
9 Maple Grove Droitwich

Worcs WR9 7QF(GB)

(72) Inventor: TAYLOR, James
9 Maple Grove Droitwich
Worcs WR9 7QF(GB)

(74) Representative: Milhench, Howard Leslie et al
R.G.C. Jenkins & Co. 26 Caxton Street
London SW1H 0RJ(GB)

(54) **Improvements in surgical stapling devices.**

(57) A surgical stapling device for use in closing incisions or wounds in skin comprises a body, a trigger member and at least one co-operating member associated with the trigger member. The staple (42) to be used for closing the skin is preformed so that when it is subsequently deformed by the at least one co-operating member when the trigger is squeezed the free ends of the staple are brought toward each other this closes the staple (42) and, if positioned correctly by the surgeon it will close the wound. The co-operating member applies a lateral deforming force to the limbs of the staple. In one embodiment the co-operating member comprises a pivotal elongate member and in another embodiment comprises a camming surface (45a,45b) defined in the body.

FIG.11

# IMPROVEMENTS IN SURGICAL STAPLING DEVICES

The invention relates generally to surgical stapling devices.

In surgical procedures conventional skin closure techniques involve the use of a needle and suture thread. Such suturing often leaves cross hatching across the scar line as the result of pressure applied to the region during the healing process. It is therefore cosmetically desirable to achieve effective suturing of a wound whilst minimising the amount of cross hatching and additional scarring in the region of the scar.

Surgical stapling devices have been proposed for use as an alternative to the conventional needle and thread skin closure techniques. Surgical stapling devices make use of staples with are surgically sterile but which are otherwise conventional in that they have a pair of substantially parallel limbs bridged by a cross-piece. The limbs are implanted in the skin on opposing sides of the wound or incision and are bent inwardly toward each other to form a loop thereby closing the wound or incision. These stapling devices are popular with surgeons because they offer a wound closing procedure which is less difficult and faster than conventional thread suturing.

However, in previously known devices the problem of scar cross hatching has not been satisfactorily solved because abnormal accumulations of fluids beneath the skin (oedema) cause wounds to swell and this causes the wound at the cross-piece of the staple to expand and gives rise to cross hatching of the scar. This swelling of the wound area can also cause difficulties in subsequently removing the staples without inflicting further trauma and causing discomfort to the patient.

British Patent Application publication No. 2,162,115 A (the teachings of which are incorporated herein by reference) discloses a skin closure device which aims to provide a solution to the above discussed problems. This skin closure device comprises a housing; a handle movably mounted relative to the housing; a former mounted in the housing so as to be movable upon movement of the handle in one direction from an inoperative limit position to an operative limit position; a mandrel disposed adjacent an outlet opening in the housing; means for supplying skin-closure staples to a location on the mandrel which is in the path of movement of the former in said one direction; said former and said mandrel being shaped so that when the device is used to close an incision in the skin, the cross-piece of the staple is arched above the incision so as to ensure that there is an air gap provided between the incision and the closure element. The skin closure device thus is arranged to implant staples in such a manner that a portion of the staple, ie. the bridging cross-piece portion above mentioned, is raised above the skin surface to create an arched air gap which accommodates swelling of the wound during healing. Whilst this device offers a solution to the abovementioned problems it can still cause distress in the area of the wound because of the mandrel used to arch the staple after application of the staple to the wound area. The reason for this is simple and stems from the fact that the mandrel is held in position over the wound by the very staple used to close the wound and can only be removed from between the staple and the wound area by sliding the mandrel from underneath the staple. In sliding the mandrel free from the staple, the surgeon runs the risk of further damaging the wound area by pinching skin between the staple and the mandrel or by catching one side of the wound for example.

In view of the foregoing an object of the present invention is to provide an improved stapling device for suturing incisions or wounds in skin. It is also an object of the present invention to provide an improved stapling device for use in inserting a staple into skin in a manner which minimises tissue trauma inflicted in the vicinity of the inserted staple.

The present invention aims to provide a device for use in closing incisions or wounds in skin, in which device a staple to be inserted into the skin is preformed so that when it is subsequently deformed by means of cooperating members which simply apply lateral deforming forces to pinch the limbs of the staple towards each other, the ends of the limbs are urged toward each other so as to close the staple.

According to one aspect of the invention therefore there is provided a device for use in applying a staple to close an incision or wound in skin, in which device a staple to be inserted into the skin is preformed so that when it is subsequently deformed by co-operating members of the device, which co-operating members apply lateral deforming forces to the limbs of the staple, free ends of the limbs of the staple are urged toward each other so as to close the staple.

According to another aspect of the invention there is provided a surgical stapling device comprising a body; a trigger member mounted to said body; and at least one co-operating member associated with said trigger member; the co-operating member being arranged to deform a staple as it is expelled from the body of the device by actuation of the trigger member by applying a lateral force to the limbs of the staple thereby to close the staple.

The preforming of the staple may, for example, be generally such that the preformed staple takes up a shape substantially akin to that which would be obtained if a conventional surgical staple as formed into its closed condition were to be deformed by pulling apart the side limbs of the staple so as to open the nip between the two extremities of the staple, but not so much as to cause the side limbs of the staple to line up with the cross-piece of the staple. With the staple thus preformed, the nip or mouth of the staple can simply be closed by applying lateral forces to the limbs to pinch them together.

In the practice of an embodiment of the invention, to be described in greater detail hereinafter, a supporting base member may be provided to prevent excessive deformation of the cross-piece of the staple during insertion into the skin. Moreover, the invention may be embodied in a device comprising a housing and a trigger movably mounted to the housing and arranged such that actuation of the trigger by a user causes the cooperating members to apply the deforming forces to the limbs of the staple.

As will become clear from the detailed description hereinafter of embodiments of the invention, a plurality of staples may be provided on a carrier, such as a magazine, arranged to deliver a staple to a position between the cooperating members when the trigger of the device is actuated by a user.

The invention also extends to preformed staples adapted for use with a stapling device according to the present invention.

Further features and advantages, together with the abovementioned features and advantages, will become clearer from a detailed description hereinafter of embodiments of the invention.

In order that the invention may be better understood exemplary embodiments will be described hereinafter with reference to the accompanying drawings, in which:

Figure 1 is a perspective view of an embodiment of a surgical stapling device;

Figures 2, 3 and 4 are schematic detail views of a staple and cooperating members;

Figure 5 is a view of an alternative form of staple;

Figure 6 is a section view of a staple inserted in an area of skin;

Figure 7 is a side. view of a staple magazine; and

Figure 8 is an end view of the staple magazine;

Figure 9 is a schematic view of another embodiment of a surgical stapling device;

Figure 10 is an exploded perspective view of a staple compartment; and

Figure 11 is a partial section view of the device of Figure 9 showing a staple being urged from the device.

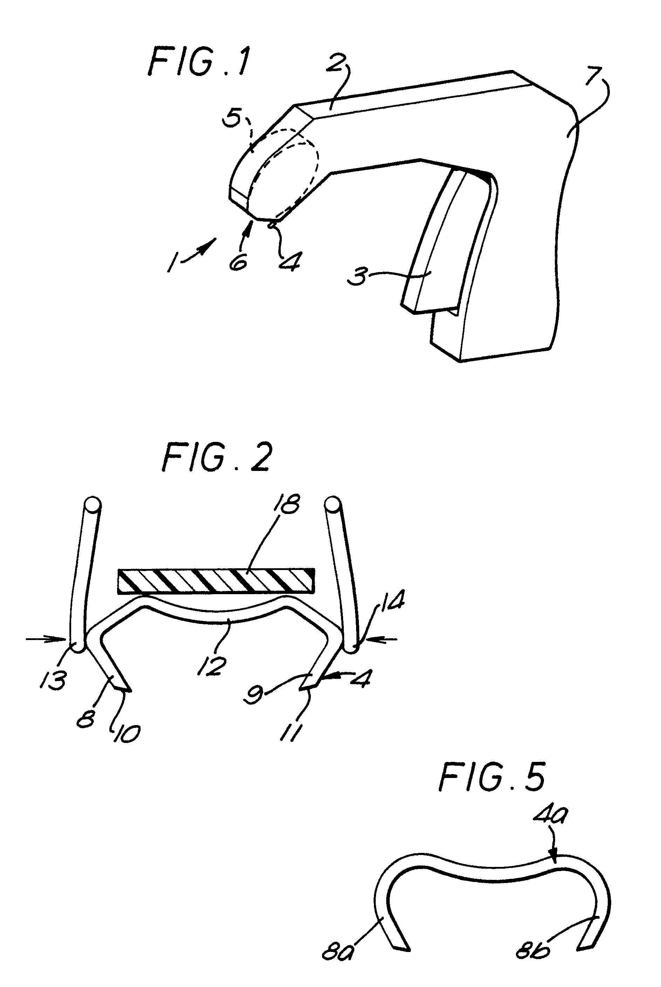

Referring firstly to Figure 1 of the accompanying drawings, a surgical skin closure or stapling device 1 comprises a housing 2 and a trigger 3 each of which can be formed from injection-moulded plastics parts. Staples, such as staple 4, are mounted on a magazine 5, shown in hidden detail as broken lines in the drawing, and are arranged on the magazine 5 to project from an opening 6 at the front of the device 1 one at a time. The trigger 3 is biassed by a biassing spring (not shown) provided in a handle portion 7 of the housing 2. With the trigger 3 in the non-actuated position shown in Figure 1, this position being limited by cooperation of the trigger 3 with the housing 2 against the force of the spring (not shown), a staple is presented at the opening 6 of the housing 2 ready for insertion into an area of skin.

As can be seen from Figures 2 to 4 of the accompanying drawings, the staple 4 for use in this embodiment is not of the conventional arrangement of two parallel limbs spaced by a bridging cross-piece, but is instead preformed to facilitate its deformation as it is being inserted into an area of skin. To this end, the staple 4 shown in Figure 2 comprises two converging limbs 8, 9 spaced by a convex (ie. with respect to the ends 10, 11 of the two limbs 8, 9) bridging cross-piece 12. An alternative form of staple 4a is shown in Figure 5 of the accompanying drawings. As can be seen from Figure 5 this form of staple 4a differs from that shown in Figure 2 in that the limbs 8a, 9a are continuously curved. It should be appreciated that both of these forms of staple are particularly suitable for use in this embodiment because, as should become clear from the further description hereinbelow, they will both readily deform in the required manner in response to lateral or side forces being applied to the two limbs of the staple. It should also be noted that the staples are not limited to the two forms shown in Figures 2 and 5 and that conventional staples may also be used following suitable modification to the device 1.

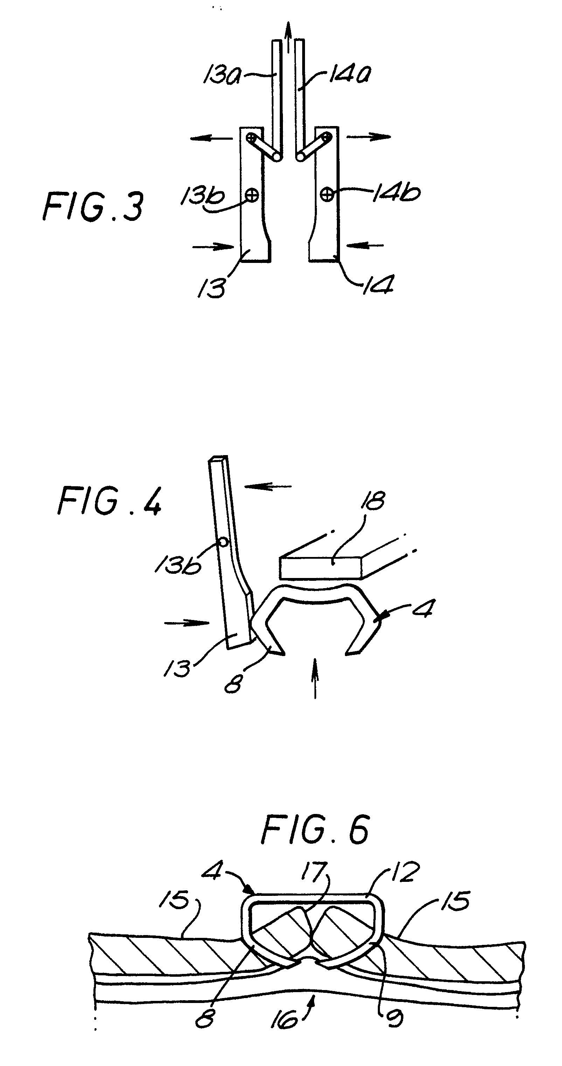

When the trigger 3 (see Figure 1) is squeezed, means (including the lever arrangement 13a, 13b and other parts not shown) cooperate with the trigger to urge end portions of two staple deforming members 13, 14 laterally towards respective limbs 8, 9 of the staple. Further pressure on the trigger results in a force being applied through the members 13, 14 to the limbs 8, 9 as the members 13, 14 pivot about respective pivot points 13b, 14b thereby urging the ends 10, 11 of the limbs toward each other and deforming the staple. The ends 10, 11 of the staple are tapered to allow for easy penetration into the skin and, as can be seen from Figure 6 of the accompanying drawings, when the staple has been fully deformed by the members 13, 14 these ends will meet or nearly meet beneath the surface 15 of the area of skin 16 to suture the wound or incision 17. It will also be noted from Figure 6 that the cross-piece 12 is straighter after insertion into the area of skin 16 than it is before. It should be appreciated that this straightening is caused by an upward force being transmitted from the members 13, 14 through the limbs 8, 9 to the cross-piece 12 and results in a gap being defined between the cross-piece 12 and the surface 15 of the skin, thereby accommodating swelling caused by oedemae and thus minimising cross hatching of the scar that will remain after the wound has healed.

In order to accommodate this straightening of the cross-piece 12 a base member 18 is provided. The base member 18 provides a substantially immovable (in relation to the forces to which it will be subjected) surface against which the straightening force delivered through the limbs 8, 9 can react and can also be arranged to provide a mount for delivering the staple to a position between the deforming members 13, 14. In this way, the base will also serve to prevent twisting or axial deformation of the staple as it is driven into an area of skin.

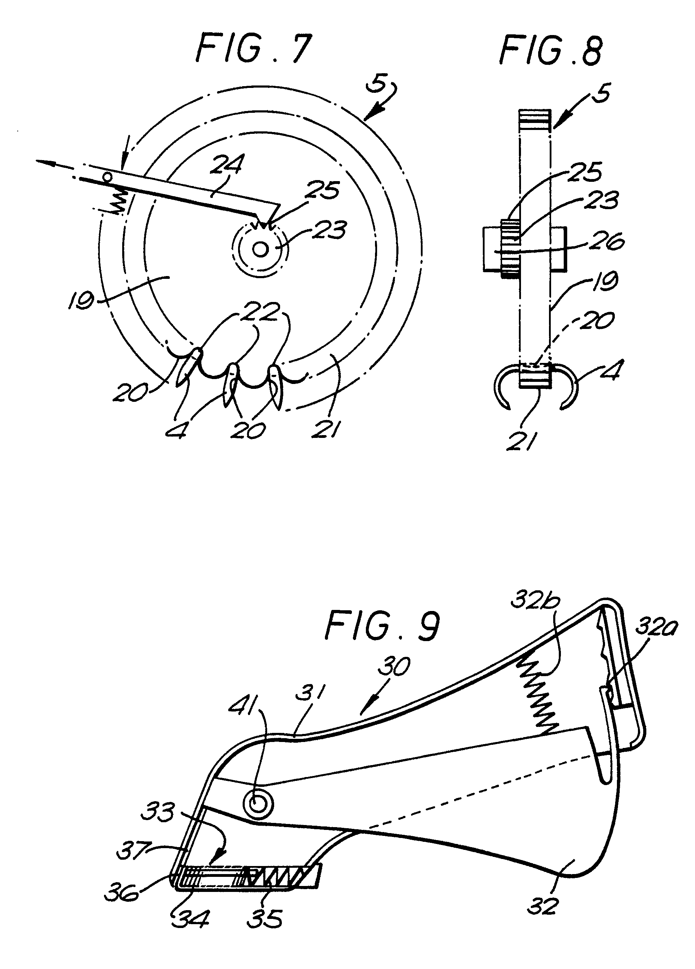

In this embodiment, the base member 18 is provided as part of the magazine 5 which is shown in Figures 7 and 8 of the accompanying drawings. Referring now to those drawings it will be seen that the magazine 5, which is made from an injection-moulded plastics material, is substantially circular in cross-section and comprises a main staple supporting wheel 19 with a plurality of staple supporting notches 20 defined in the circumferential surface 21 thereof. Each notch 20 defines a base 22 which acts as the abovementioned base member 18 to provide support for the staple during deformation. The notches 20 are also dimensioned so that one staple 4 can be supported therein by friction engagement for example. Typically the magazine 5 will be provided with thirty six notches 20 to accommodate thirty six staples 4.

A ratchet wheel 23 coaxial to the staple supporting wheel 19 is also provided and is arranged to cooperate with means (including a biassed ratchet arm 24 and other members not shown) associated with the trigger 3 for rotation of the magazine to present the next staple at the opening 6 at the front of the device (see Figure 1) once the previous staple has been used. In order to simplify construction of the device the ratchet wheel 23 may include the same number of ratchet teeth 25 as there are staples, for example thirty six teeth. The magazine 5 also includes a hub 26 which is arranged to engage rotatingly with a journal or spindle (not shown) in the housing 2.

The principal features of a second embodiment of the invention are shown in Figures 9 to 11 of the accompanying drawings. Referring now to Figure 9 of the drawings it can be seen that this stapling device comprises a housing 31 and a trigger member 32 each of which can be formed as injection moulded plastics parts. The housing includes a compartment, indicated generally at 33, adapted to receive a plurality of staples 34. The staples 34 are urged forward in the compartment by way of a biassing member such as a spring 35 so that the forwardmost staple is positioned over an opening 36 to one end of the compartment 34. In this position the forwardmost staple is placed between two limbs of a staple urging member 37 which is in turn coupled to the trigger member 32. The trigger member 32 may include a ratchet arrangement 32a including a biassing spring 32b to facilitate use of the device 30.

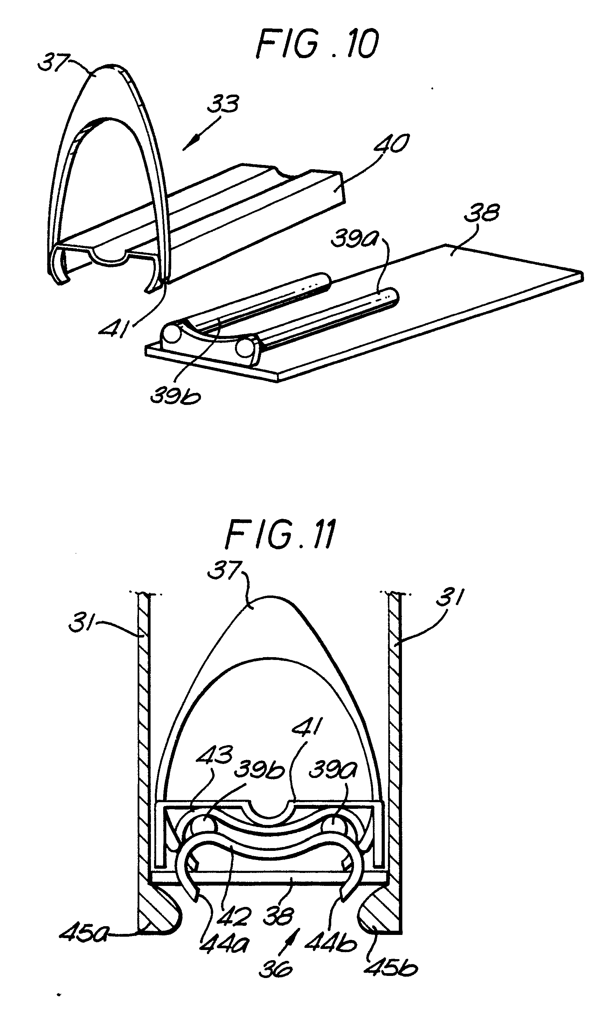

As can be seen from Figure 10 of the accompanying drawings, the compartment 33 is formed from a lower support member 38 comprising two guiding rails 39a, 39b which in use serve to guide the staples toward the open end of the compartment. The compartment also comprises an upper cover 40 which is positioned over the lower member 38 such that guide tracks 41 formed therein will guide the staple urging member 37 toward the opening. The compartment 33 may be supplied as part of the device 30 with the device 30 being intended to be disposed of once all staples have been used or may be arranged to be accessible so that new staples can be placed therein when required. Alternatively the compartment 33 may be supplied as a replaceable cartridge which can be replaced with a new cartridge when the store of staples therein is exhausted.

When the trigger member 32 is squeezed it will rotate about a pivot part 41 (see Figure 9) thereby driving the staple urging member 37 downwardly. As can be seen from Figure 11 of the accompanying drawings, this movement of the urging member 37 causes the forwardmost staple 42 to be urged downwardly away from other staples 43 in the compartment 33 and toward the opening 36. As the staple 42 is urged out through the opening 36, its side limbs 44a, 44b contact respective deforming cams 45a, 45b provided on the side walls of the housing in the vicinity of the opening 36. The effect of these deforming cams 45a, 45b is to cause the ends of the limbs 44a, 44b to move toward each other in a manner similar to that previously described herein in respect of the first embodiment such that when the staple 42 is fully exited from the opening 36 the ends 44a, 44b will meet or nearly meet beneath the surface of an area of skin to which the staple 42 has been applied in much the same way as that illustrated in Figure 6 of the accompanying drawings.

It will be appreciated that this embodiment too offers the advantage of minimising tissue trauma by providing a means for deforming the staple without the need for a mandrel.

It is envisaged that the above described embodiments of stapling devices may be supplied as a disposable item where, once all the staples in the magazine or compartment have been used, the device is simply thrown away. There are advantages to be gained in terms of hygiene in providing a disposable device but, whilst the cost of individual devices can be kept down by designing them to be disposable, the overall operating cost may be increased by adopting this approach. It is therefore also envisaged that a reusable device in which only exhausted magazines are replaced may also be made available.

Embodiments of the invention have been described and it will be clear to those possessed of the appropriate skills that many modifications are possible. All such modifications are intended to fall within the spirit and scope of the invention.

**Claims**

1. A device for use in applying a staple to close an incision or wound in skin, in which device a staple to be inserted into the skin is preformed so that when it is subsequently deformed by co-operating members of the device, which co-operating members apply lateral deforming forces to the limbs of the staple, free ends of the limbs of the staple are urged toward each other so as to close the staple.

2. A device as claimed in claim 1, wherein the co-operating members comprise staple deforming members arranged to pivot laterally toward respective limbs of the staple,

3. A device as claimed in claim 1, wherein the co-operating members comprise deforming cam portions formed in the body of the device and arranged to define a restricted aperture such that when a staple is urged therethrough the free ends of the limbs of the staple move toward each other.

4. A device as claimed in any preceding claim, wherein the staple is urged into a position in which it is deformed by way of a trigger member.

5. A device as claimed in any preceding claim, wherein a plurality of staples are provided in an annular cartridge arranged to be mounted to said device such that one staple at a time is presented for application.

6. A device as claimed in any of claims 1 to 4, wherein a plurality of staples are provided in a compartment in the device and are urged to a position for application by way of a resilient biassing member.

7. A surgical stapling device comprising:
a body;

a trigger member mounted to said body; and at least one co-operating member associated with said trigger member; the co-operating member being arranged to deform a staple as it is expelled from the body of the device by actuation of the trigger member by applying a lateral force to the limbs of the staple thereby to close the staple.

8. A device as claimed in claim 7, wherein the at least one co-operating member comprises a pivotal elongate member coupled to said trigger member for actuation thereby.

9. A device as claimed in claim 7, wherein the at least one co-operating member comprises a camming surface which defines a restriction through which the staple is urged on actuation of the trigger member.

10. A preformed staple adapted for use with a device as claimed in any preceding claim.

FIG.1

FIG.2

FIG.5

FIG.3

13a    14a

13b    14b

13    14

FIG.4

18

13b

4

13

8

16

FIG.6

4    17    12

15    15

8    16    9

# FIG.7

# FIG.8

# FIG.9

FIG .10

FIG .11